# EUROPÄISCHE PATENTANMELDUNG

(11) **EP 0 613 880 A1**
(43) Veröffentlichungstag der Anmeldung: **07.09.1994**
(21) Anmeldenummer: 94100906.0
(22) Anmeldetag: 22.01.1994
(51) Int. Cl.: C07C 217/48, C07C 217/76, C07C 217/10, C07C 33/44, C07C 43/178, C09B 11/22, G01N 33/58, G01N 33/532, G01N 33/94

(54) **Mittel und Verfahren zur immunologischen Bestimmung von Diphenhydramin und dessen Metaboliten**

(30) Priorität: 04.03.1993 DE 4306697
(71) Anmelder: MERCK PATENT GmbH, D-64293 Darmstadt (DE)
(72) Erfinder: Jalalian, Mohammad, Dr., D-64354 Reinheim (DE); Schönefeld, Ulrich, Dr., D-64287 Darmstadt (DE)

(57) **Zusammenfassung**

Die Erfindung betrifft Mittel und Verfahren zur immunologischen Bestimmung von Diphenhydramin in Körperflüssigkeiten sowie die für eine Bestimmung erforderlichen Tracer, Immunogene und Antikörper und deren Herstellung. Als Tracer und Immunogene werden Verbindungen der allgemeinen Formel (I) eingesetzt 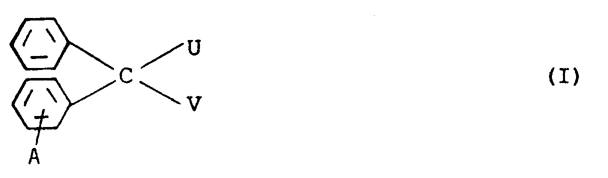
worin
- U =: H oder W,
- V =: X-(CH₂)ₙ-Y oder X-W,
- A =: [(CH₂)ₖ-X-(Q)ₘ-Z]ₘ,
- W =: (CH₂)ₙ-(Q)ₘ-Z,
- X =: O, S, NR,
- Y =: COOR, NR₂,
- Q =: Crosslinker oder Spacer,
- Z =: Marker oder Trägerstoff
- R =: H oder Alkyl mit 1-8 C-Atomen,
- n =: 1-10,
- m =: 0 oder 1,
- k =: 0-4,
bedeuten, und der Substituent A nur dann vorhanden ist, wenn U = H und V = X-(CH₂)ₙ-Y ist.

## Beschreibung

Die Erfindung betrifft Mittel und Verfahren zur immunologischen Bestimmung von Diphenhydramin und dessen Metaboliten in Körperflüssigkeiten oder in vorbehandelten Körperflüssigkeiten sowie die für eine Bestimmung erforderlichen Tracer, Immunogene und Antikörper und deren Herstellung.

Arzneimittel werden häufig unter- oder überdosiert verabreicht und mißbräuchlich verwendet. Diese Erfahrung erfordert in zunehmendem Maße die Messung der Konzentration der Arzneimittel in Körperflüssigkeiten wie Serum, Plasma, Speichel oder Urin. Besonders geeignete und schnelle Bestimmungsverfahren sind Immunoassays, insbesondere Fluoreszenzpolarisations-Immunoassays oder Enzym-Immunoassays.

Diphenhydramin (2-Diphenylmethoxy-N,N'-dimethylethanamin) gehört zu den H1-Antihistaminika. Wie die meisten Antihistaminika besitzt auch Diphenhydramin als weitere Wirkung einen sedierenden, hypnotischen Effekt. Diphenhydramin wird auch als Hypnotikum in den Handel gebracht. Nachdem nahezu alle anderen Schlafmittel der Rezeptpflicht unterstellt worden sind, wird das frei verkäufliche Diphenhydramin sehr häufig überdosiert und mißbraucht. Die wichtigste Nebenwirkung ist die Beeinflussung des Zentralnervensystems und damit die Einschränkung des Reaktionsvermögens. (Überdosierung kann zu Atemlähmung und Herzversagen führen.

Die Bestimmung von Diphenhydramin erfolgte bisher durch RIA- oder HPLC-Methoden. Diese Methoden erfordern jedoch eine Probenvorbehandlung bzw. Derivatisierung und ein aufwendiges analytisches Instrumentarium; sie sind darüberhinaus sehr zeitaufwendig.

Für die Herstellung eines Immunoassays benötigt man bekanntlich Tracer, Immunogene und spezifische Antikörper. Nach den im Stand der Technik beschriebenen Methoden wird ein Tracer durch Kopplung eines zu bestimmenden Analyten oder dessen Derivat mit einem Marker (Chromogen, Fluorogen, Luminogen, Enzym, radioaktiv markierte Verbindung) hergestellt. Ein Immunogen wird durch Kopplung eines Haptens oder Haptenderivats mit einem Trägerstoff hergestellt. Die Kopplungsreaktionen erfolgen in der Regel mit Hilfe eines Crosslinkers oder Spacers. Immunogene werden in einem wäßrigen System, vorzugsweise in einem Puffersystem hergestellt und anschließend aufgereinigt.

Der Erfindung liegt die Aufgabe zugrunde, Mittel und Verfahren zur Verfügung zu stellen, die es erlauben, Diphenyhdramin und dessen Metabolite ohne großen analytischen Aufwand schnell und reproduzierbar zu bestimmen.

Gegenstand der Erfindung sind Verbindungen der allgemeinen Formel (I) 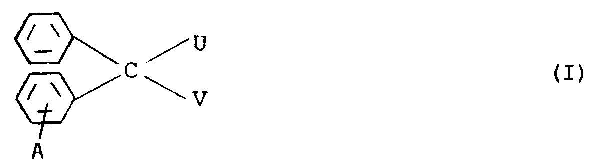
worin
- U =: H oder W,
- V =: X-(CH₂)ₙ-Y oder X-W,
- A =: [(CH₂)ₖ-X-(Q)ₘ-Z]ₘ,
- W =: (CH₂)ₙ-(Q)ₘ-Z,
- X =: O, S, NR,
- Y =: COOR, NR₂,
- Q =: Crosslinker oder Spacer,
- Z =: Marker oder Trägerstoff
- R =: H oder Alkyl mit 1-8 C-Atomen,
- n =: 1-10,
- m =: 0 oder 1,
- k =: 0-4,
bedeuten, und der Substituent A nur dann vorhanden ist, wenn U = H und V = X-(CH₂)ₙ-Y ist.

Ein weiterer Gegenstand der Erfindung sind Antikörper, die durch Immunisierung mit einem Immunogen der allgemeinen Formel (I) erhältlich sind, worin Z ein Trägerstoff bedeutet.

Ferner betrifft die Erfindung ein Mittel zur immunologischen Bestimmung von Diphenhydramin und dessen Metaboliten in Körperflüssigkeiten, das dadurch gekennzeichnet ist, daß es
a) ein markiertes Diphenhydraminderivat der allgemeinen Formel (I), worin Z ein Marker bedeutet,
b) Antikörper, die Diphenhydramin, dessen Metabolite und das markierte Diphenhydraminderivat binden können und
c) gegebenenfalls ein Enzymsubstrat enthält.

Ein weiterer Gegenstand der Erfindung ist ein Verfahren zur immunologischen Bestimmung von Diphenhydramin und dessen Metaboliten in Körperflüssigkeiten durch Inkubation der zu untersuchenden Probe mit einem markierten Diphenhydraminderivat der allgemeinen Formel (I), worin Z ein Marker bedeutet, und Antikörper, die Diphenyhdramin, dessen Metabolite und das markierte Diphenhydraminderivat binden können sowie gegebenenfalls mit einem Enzymsubstrat und Messung der Fluoreszenz oder der Enzymaktivität.

Der Tracer ist bei einem Fluoreszenzpolarisations-Immunoassay mit einer fluoreszeierenden Verbindung, bei einem Enzym-Immunoassay mit einem Enzym markiert, wobei der Marker gegebenenfalls über einen sog. Crosslinker gebunden ist.

Bevorzugte Verbindungen der allgemeinen Formel (I) sind solche der nachstehenden Formeln (Ia), (Ib) und (Ic), insbesondere solche der Formel (Ia). 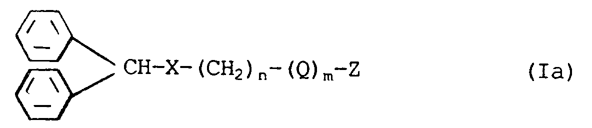
worin
- X =: O, S, NR,
- Q =: Crosslinker oder Spacer,
- Z =: Marker oder Trägerstoff,
- R =: H oder Alkyl mit 1-4 C-Atomen,
- n =: 1-10,
- m =: 0 oder 1
bedeuten,
der Formel (Ib) 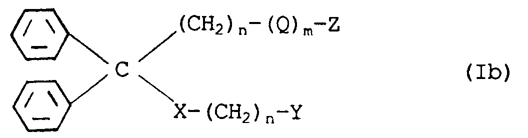
worin x, Q, Z, n und m die oben angegebene Bedeutung haben und
- Y =: COOR, NR₂,
- R =: H oder Alkyl mit 1-8 C-Atomen
bedeuten, und der Formel (Ic) 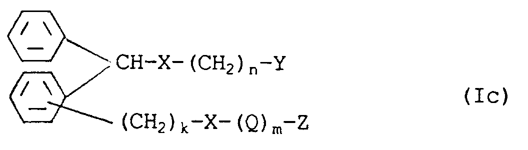
worin X, Q, Z, Y, n, m und k die oben angegebene Bedeutungen haben.

Die Herstellung der Verbindungen der allgemeinen Formel (I) erfolgt in der Weise, daß man z.B. ausgehend von Benzhydrol und Halogencarbonsäuren, deren Estern oder Nitrilen die entsprechenden Ausgangsverbindungen herstellt, die auch durch Reduktion in die entsprechenden Alkohole oder Amine überführt werden können. Die so erhaltenen Diphenhydraminderivate werden gegebenenfalls mit Hilfe von Crosslinkern oder Spacern an einen Marker (Fluoreszeinderivate, Enzyme) bzw. Trägerstoff gekoppelt.

Geeignete Fluoreszeinderivate sind z.B. 4-Aminomethylfluoreszein, 4-Aminofluoreszein, Fluoreszeinisothiocyanat, Carboxyfluoreszein, 2,4-Dichlor-1,3,5-triazin-2-ylaminofluoreszein, 4-Chlor-6-methoxy-1,3,5-triazin-2-ylaminofluoreszein, vorzugsweise 4-Aminomethylfluoreszein.

Geeignete Crosslinker für die Kopplung des Fluoreszeinderivats an das Diphenhydraminderivat sind aus der Literatur bekannt. Bevorzugte Crosslinker für das erfindungsgemäße Verfahren sind z.B. 1-Ethyl-3-dimethylaminopropylcarbodiimid-hydrochlorid (EDC), N-Hydroxysuccinimid (NHS), N-(3-dimethylaminopropyl)-N'-ethylcarbodiimid-hydrochlorid (ECD), Dicyclohexylcarbodiimid (DCC), Chlorameisensäureester.

Die Herstellung der Tracer erfolgt nach bekannten Methoden, wobei die Diphenhydraminderivate über ihre reaktiven Gruppen auch direkt, d.h. ohne die Verwendung eines Crosslinkers, an geeignete Derivate der Fluoreszenzfarbstoffe oder Enzyme kovalent gekoppelt werden können. Die Reaktion wird vorzugsweise in einem alkalischen Medium, z.B. in Methanol/Triethylamin durchgeführt. Die Aufreinigung des Tracers erfolgt mittels Dünnschichtchromatographie.

Die für die erfindungsgemäße Bestimmung von Diphenhydramin erforderlichen spezifischen Antikörper werden durch Immunisierung mit einem Immunogen aus einem Diphenhydraminderivat, das über einen Crosslinker an einen Trägerstoff kovalent gekoppelt ist, erhalten. Als Crosslinker eignen sich die oben für die Tracerherstellung genannten Verbindungen. Als Trägerstoffe kommen Polysaccharide und deren Derivate, Lipopolysaccharide und deren Derivate, Polypeptide und Proteine, vorzugsweise z.B. Rinderserumalbumin (BSA), Hemocyanin, IgG, Keyholelimpethemocyanin (KLH), Ovalbumin, Thyroglobulin oder Lactalbumin infrage. Das Immunogen wird nach bekannten Methoden in wäßriger Lösung hergestellt.

Zur Herstellung der erforderlichen Antikörper wird das aufgereinigte Immunogen in üblicher Weise in zwei- bis dreiwöchigem Abstand in bestimmte Tiere (Schafe, Ziegen, Mäuse, Kaninchen) injiziert. Nach 3 bis 6 Immunisierungen haben sich die gewünschten Antikörper gebildet. Die Aufarbeitung und Reinigung der Antikörper erfolgt nach bekannten Methoden.

Der Tracer für den Enzymimmunoassay wird aus einem Diphenhydraminderivat und einem Markerenzym, z.B. Alkalische Phosphatase, Glucosidase, Galactosidase, Peroxidase, hergestellt. Die Synthese erfolgt in Gegenwart der für die Immunogensynthese genannten Lösungsmitteln, wobei auf den Crosslinker auch verzichtet werden kann.

Das erfindungsmäßige Verfahren zur Bestimmung von Diphenhydramin und dessen Metaboliten, wie z.B. Diphenylmethoxyessigsäure und deren Konjugate, wird so durchgeführt, daß die mit Puffer verdünnte oder auch die unverdünnte Probelösung mit dem Tracer und dem Antikörper gemischt werden. Nach einer bestimmten Inkubationszeit wird die Konzentration von Diphenhydramin und dessen Metaboliten direkt in der Reaktionslösung bestimmt.

Im Falle des Fluoreszenzpolarisations-Immunoassay wird der Tracer z.B. mit blauem polarisiertem Licht (488 nm) angeregt; die Polarisation des grünen ausgestrahlten Lichts (532 nm) hängt von der Größe und der Gestalt des Tracers ab. Die Polarisation des ausgestrahlten Lichts steigt an, wenn der Tracer an den spezifischen Antikörper gebunden vorliegt. Fluoreszenzpolarisations-Immunoassays sind kompetitive Immunoassays, d.h. Tracer und Analyt konkurrieren um die Bindungsstelle am Antikörper. Das Signal der Fluoreszenzpolarisation hängt umgekehrt proportional von der Konzentration des Analyten in der Probe ab. Ist z.B. die Konzentration des Analyten in der Probe hoch, bindet nur eine kleine Menge des Tracers an den Antikörper und die Fluoreszenzpolarisation ist niedrig.

Im Falle des kompetitiven Enzym-Immunoassays wird der spezifische Antikörper auf einem festen Träger, z.B. auf Mikrotiterplatten oder in Röhrchen, immobilisiert und mit der Probelösung und dem Enzymkonjugat inkubiert. Anschließend wird der Träger gewaschen, mit dem entsprechenden Enzymsubstrat versetzt und die Konzentration des Analyten photometrisch bestimmt.

### Beispiel 1

a) 0,48 g (21 mmol) Natrium werden in 10 ml abs. Ethanol unter Luftfeuchtigkeitsausschluß gelöst und mit 1,8 g (9,8 mmol) Benzhydrol umgesetzt. Anschließend werden 0,95 g (10 mmol) Chloressigsäure zu der Lösung zugegeben und über Nacht unter Rückfluß erhitzt. Nach dem Abkühlen wird das Lösungsmittel im Vakuum entfernt und der Ansatz in 3N Natronlauge aufgenommen und mit Ether ausgeschüttelt. Die Etherphase wird verworfen; die wäßrige Phase wird mit verd. Salzsäure angesäuert und wieder mit Ether ausgeschüttelt. Nach der Trocknung des Lösungsmittels über Natriumsulfat wird Diphenylmethoxyessigsäure aus der Etherlösung isoliert.
b) 61 mg (1,6 mmol) Lithiumaluminiumhydrid werden in 20 ml abs. Ether suspendiert. Unter Luftfeuchtigkeitsausschluß und Rückflußkühlung werden 480 mg (2 mmol) Diphenylmethoxyessigsäure, gelöst in 5 ml abs. Ether, zu der Suspension langsam zugetropft. Nach Beendigung des Zutropfens wird der Ansatz 2 Stunden erhitzt. Anschließend wird die Lösung abgekühlt und vorsichtig mit Eiswasser versetzt. Nach Zugabe von 10%iger Schwefelsäure wird der Niederschlag aufgelöst. Die organische Phase wird getrennt und die wäßrige Phase noch 3mal mit Ether ausgeschüttelt. Die vereinigten organischen Phasen werden über Natriumsulfat getrocknet und das Lösungsmittel im Vakuum entfernt. Das erhaltene Diphenylmethoxyethanol wird säulenchromatographisch gereinigt.

### Beispiel 2

a) 0,48 g (21 mmol) Natrium werden in 10 ml abs. Ethanol unter Luftfeuchtigkeitsausschluß gelöst und mit 1,8 g (9,8 mmol) Benzhydrol umgesetzt. Anschließend werden 1,19 g (10 mmol) Bromacetonitril zu der Lösung zugegeben und 16 h unter Rückfluß erhitzt. Nach Beendigung der Reaktion wird das Lösungsmittel im Vakuum entfernt. Der Rückstand wird in 20 ml Ether aufgenommen und mit Natriumhydroxidlösung und Wasser gewaschen. Nach dem Trocknen über Natriumsulfat und Entfernung des Lösungsmittels wird das erhaltene Diphenylmethoxyacetonitril isoliert.
b) 46 mg (1,2 mmol) Lithiumaluminiumhydrid werden in 10 ml abs. Ether suspendiert. Unter Luftfeuchtigkeitsausschluß werden 446 mg (2 mmol) Diphenylmethoxyacetonitril, gelöst in 5 ml abs. Ether, zu der Suspension langsam zugetropft. Anschließend wird der Ansatz 16 Stunden unter Rückfluß erhitzt. Nach Beendigung der Reaktion werden 2 ml Ethylacetat und 1 ml Wasser, das mit Natriumchlorid gesättigt ist, zugetropft und anschließend mit 20 ml Dioxan verdünnt und abfiltriert. Das Filtrat wird über Natriumsulfat getrocknet und das Lösungsmittel entfernt. Das erhalten Diphenylmethoxy-2-ethylamin wird säulenchromatographisch gereinigt.

### Beispiel 3

a) 0,48 g Natrium werden in 10 ml abs. Ethanol unter Luftfeuchtigkeitsausschluß gelöst und mit 1,8 g Benzhydrol umgesetzt. Man gibt 1,67 g Bromessigsäureethylester zu der Lösung und erhitzt über Nacht unter Rückfluß. Nach Beendigung der Reaktion wird das Lösungsmittel im Vakuum entfernt und das verbleibende Öl in Ether aufgenommen. Die Etherphase wird mit kalter Natriumhydroxidlösung und anschließend mit Wasser gewaschen. Nach dem Trocknen über Natriumsulfat und Entfernung des Lösungsmittels erhält man Diphenylmethoxyessigsäureethylester als öliges Produkt.
b) 10 ml abs. Acetonitril werden mit 0,15 g Natriumhydrid und anschließend mit 1,2 g Diphenylmethoxyessigsäureethylester unter Kühlen und Rühren versetzt. Wenn die Gasentwicklung abgeklungen ist, fügt man 0,6 g Bromacetonitril zu und rührt bei Raumtemperatur über Nacht. Nach Beendigung der Reaktion wird das Lösungsmittel im Vakuum entfernt und der Niederschlag in Ether aufgenommen. Die Etherphase wird mit 2%iger Natriumhydroxidlösung und Wasser gewaschen und über Natriumsulfat getrocknet. Das Lösungsmittel wird im Vakuum entfernt und man erhält 1,1-Diphenyl-1-acetonitril-methoxysssigsäureethylester als öliges Produkt.
c) 92 mg Lithiumaluminiumhydrid werden in 20 ml abs. Ether suspendiert. Unter Luftfeuchtigkeitsausschluß werden 1,1 g 1,1-Diphenyl-1-acetonitril-methoxyessigsäureethylester, gelöst in 5 ml abs. Ether, zu der Suspension langsam zugetropft. Anschließend wird der Ansatz über Nacht unter Rückfluß erhitzt. Nach Beendigung der Reaktion werden 2 ml Ethylacetat und 1 ml Wasser, welches mit Natriumchlorid gesättigt ist, zugetropft und mit 20 ml Dioxan verdünnt und abfiltriert. Das Filtrat wird über Natriumsulfat getrocknet und das Lösungsmittel im Vakuum entfernt. Man erhält einen braunen Niederschlag von 1,1-Diphenyl-1-ethylamin-methoxyessigsäureethylester.
d) 1 g 1,1-Diphenyl-1-ethylamin-methoxyessigsäureethylester in Ethanol wird in einer 5%igen Natriumhydroxidlösung 2 h unter Rückfluß erhitzt. Nach Abkühlen wird das Ethanol entfernt und der Rückstand in Wasser aufgenommen. Die wäßrige Phase wird mit 2 N Salzsäure unter Kühlung neutralisiert und auf pH 4-5 gebracht. Anschließend wird das Wasser im Vakuum eingeengt. Es fällt ein weißer Niederschlag aus, der nach Abkühlen abfiltriert und mit Eiswasser gewaschen wird. Die erhaltene 1,1-Diphenyl-1-ethylamin-methoxyessigsäure wird säulenchromatographisch gereinigt.

### Beispiel 4

a) Aus 3,16 g (0,13 mol) Magnesiumspänen, 55 ml Ether und 13,65 ml (0,13 mol) Brombenzol wird eine Grignardverbindung hergestellt. 20 g (0,13 mol) 3-Nitrobenzaldehyd werden in 300 ml Toluol gelöst, auf -70 °C gekühlt (MeOH/Trockeneis) und während 1,5 h die Grignardverbindung zugetropft. Am Ende gibt man wenig Ethanol zu, gießt auf verd. Salzsäure und schüttelt aus. Die organische Phase wird über Magnesiumsulfat getrocknet und über eine Kieselgelsäule chromatographiert (350 g Flash-Kieselgel, eluiert mit 1,5 l Toluol, 1 l Tol/Ether (99:1) und 1,5 l Tol/Ether (97:3)). Die produkthaltigen Fraktionen werden im Vakuum eingeengt und man erhält 22 g 3-Nitrobenzhydrol als gelbes Öl.
b) 2,2 g (10 mmol) 3-Nitrobenzhydrol werden in Toluol gelöst und mit 1,15 g (15 mmol) Kaliumtert.-butylat bei Raumtemperatur 1 h gerührt. Nach Zugabe von 1,1 g (ca. 10 mmol) 1-Chlor-2-dimethylamino-ethan wird die Lösung bei 70 °C 18 h gerührt. Nach Beendigung der Reaktion wird die Reaktionslösung mit Salzsäure/Wasser (pH = 1) ausgeschüttelt und die wäßrige Phase 1mal mit Toluol/Ether und 1mal mit Ether extrahiert. Die organische Phase wird verworfen. Die wäßrige Phase wird mit 5 N Natronlauge auf pH 12 eingestellt und 3mal mit Ether extrahiert. Die Etherphase wird über Natriumsulfat getrocknet und das Lösungsmittel im Vakuum entfernt. Man erhält Dimethylaminoethyl(m-nitro)benzhydrolether als öliges Produkt.
c) 3 g (10 mmol) Dimethylaminoethyl(m-nitro)benzhydrolether werden mit 7,8 g (32,5 mmol) Na₂S · 9 H₂O und 3,6 g (64 mmol) NaHS sowie 60 ml Wasser und 30 ml Ethanol versetzt und 1 h unter Rückfluß gekocht. Nach dem Abkühlen wird mit Ether versetzt, 2mal ausgeschüttelt, über Magnesiumsulfat getrocknet und im Vakuum eingeengt. Zur weiteren Reinigung wird über eine Kieselgelsäule chromatographiert (Lösungsmittel Petrolether/Triethylamin (9:1), mit Zusatz von 1-5 % Methanol). Man erhält 2,6 g (96 %) Dimethylaminoethyl(m-amino)benzhydrolether als gelbes Öl.

### Beispiel 5

### Herstellung eines fluoreszenz-markierten Tracers

Die gemäß Beispiel 1 hergestellte Diphenylmethoxyessigsäure wird mittels der Carbodiimid-Methode mit Aminomethylfluoreszein umgesetzt. Als Reaktionsmedium dient eine 1:1-Mischung von Pyridin und N,N-Dimethylformamid. Die Reinigung und Charakterisierung erfolgt dünnschichtchromatographisch.

Im einzelnen werden dabei 10 mg N-(3-Dimethylaminopropyl-N'-ethylcarbodiimid-hydrochlorid (EDC), 5 mg N-Hydroxysuccinimid (NHS), 5 mg der Diphenylmethoxyessigsäure und 3,5 mg 5-(Aminomethyl)-fluoreszein in 250 µl Reaktionsmedium gelöst. Der Ansatz wird bei Raumtemperatur unter Lichtschutz inkubiert. Nach 3 Tagen wird der Ansatz auf Dünnschicht-Fertigplatten Kieselgel 60 aufgetragen. Als Laufmittel dient eine 45:4:1-Mischung aus Ethylacetat` Methanol und Essigsäure. Die Entwicklungszeit beträgt ca. 1 Stunde. Das Produkt weist einen Rf-WErt von ca. 0,9 auf. Es wird zusammen mit dem Kieselgel aus vom Träger gekratzt und mit Methanol eluiert.

### Beispiel 6

### Herstellung eines enzym-markierten Tracers

57 mg N-(3-Dimethylaminopropyl)-N'-ethylcarbodiimid-hydrochlorid (EDC) und 38 mg N-Hydroxysuccinimid (NHS) werden in 500 µl demineralisiertem Wasser gelöst und mit 230 mg Diphenylmethoxyessigsäure, gelöst in 1,5 ml demineralisiertem Wasser, versetzt. 50 mg Meerrettichperoxidase werden in 25 ml demineralisiertem Wasser gelöst. Beide Lösungen werden vereinigt und 3 Stunden bei Raumtemperatur gerührt. Der Ansatz wird dann bei 4 °C gegen Phosphatpuffer dialysiert.

### Beispiel 7

### Herstellung des Antikörpers gegen Diphenhydramin und dessen Metaboliten

0,8 mg eines Immunkonjugats aus einem Diphenhydraminderivat und BSA werden mit einem Adjuvans (komplettes oder inkomplettes Freund'sches Adjuvans, Aluminiumhydroxid, Wasser-Öl-Emulsion oder Muramyldipeptid) emulgiert. Mit dieser Emulsion wird das Tier zweimal im Abstand von 21 Tagen immunisiert. Danach wird es 4-12mal mit einer Emulsion aus 0,8 mg des Immunkonjugats mit inkomplettem Freund'schen Adjuvans im Abstand von 21 Tagen immunisiert. Die Antikörper werden in üblicher Weise isoliert und aufgereinigt.

### Beispiel 8

### Fluoreszenzpolarisations-Immunoassay

Der Test wird in der Regel bei 37 °C Inkubationstemperatur durchgeführt. Zur Messung der Fluoreszenzpolarisation wird ein Fluorimeter mit Polarisations-Einrichtung oder ein speziell für derartige Messungen konstruiertes Analysengerät eingesetzt. Als Reagenz 1 dient das verdünnte Antiserum in gepufferter Lösung; als Reagenz 2 eine gepufferte Tracerlösung. Zur Stabilisierung und Verhinderung mikrobiellen Bewuchses können beide Reagenzien Stabilisatoren wie Proteine, Detergenzien, Azid enthalten.

10 µl der Probe bzw. der Standard-Lösung werden mit 1 ml Reagenz 1 vermischt. Nach Vorinkubation wird der Probenleerwert gemessen. 50 µl Reagenz 2 (Tracer) werden zugefügt und nach einer festgelegten Inkubationszeit der Probenwert gemessen. Mit der so bestimmten Polarisation kann anhand einer Eichkurve die in der Probe vorliegende Konzentration des Analyten bestimmt werden. Für Gruppentests und dem Nachweis von Drogenmißbrauch ist in der Regel eine qualitative bzw. halbquantitative Aussage ausreichend. Hierbei wird der Test anhand einer sogenannten cut-off-Kontrolle mit einem definierten Schwellenwert kalibriert. Liegt die gemessene Polarisation der Probe unterhalb der cut-off-Kontrolle, so wird die Probe als positiv bewertet.

Für geeignete Antikörper/Tracer-Paare ist es zweckmäßg, Antikörper und Tracer in einem Reagenz zu vereinigen. Falls eine Leerwertmessung notwendig ist, etwa wegen der Gefahr einer bewußten Verfälschung der Probe durch den Probanden, kann diese anhand einer mit Pufferlösung verdünnten Probe erfolgen.

### Beispiel 9

### Enzym-Immunoassay

Die Bestimmung erfolgt bei Raumtemperatur nach dem Prinzip des kompetitiven Enzym-Immunoassays. Als feste Phase kommen Mikrotiterplatten oder Röhrchen zur Anwendung. Je nach Probenmaterial kann eine Enteiweißung oder Vorverdünnung erforderlich sein. Der Antikörper wird auf der festen Phase direkt oder indirekt (über anti-Schaf-IgG) immobilisiert. Im Mikrotiterplattenformat werden pipettiert:
50 µl native, vorbehandelte oder verdünnte Probe bzw. Kalibrator
50 µl Enzym-markierter Tracer aus Beispiel 6
Nach einer Inkubationszeit von einer Stunde bei Raumtemperatur wird die Festphase mehrfach gewaschen. Dann wird ein Peroxidasesubstrat zugegeben:
100 µl 2,2'-AzinobiS(3-ethylbenzthiazolin-6-sulfonsäure)Diammoniumsalz (2 mmol/l) und Perborat (1,5 mmol/l) in Citratpuffer, pH 4,5.

Nach einer Inkubationszeit von 15 Minuten wird die optische Dichte bei einer Wellenlänge von 405 nm gemessen. Mit der so bestimmten optischen Dichte kann anhand einer Eichkurve die in der Probe vorliegende Konzentration des Analyten bestimmt werden. Für Gruppentests und dem Nachweis von Drogenmißbrauch ist in der Regel eine qualitative bzw. halbquantitative Aussage ausreichend. Hierbei wird der Test anhand einer sogenannten cut-off-Kontrolle mit einem definierten Schwellenwert kalibriert. Liegt die gemessene optische Dichte der Probe unterhalb der der cut-off-Kontrolle, so wird die Probe als positiv bezeichnet.

## Patentansprüche

1. Verbindungen der allgemeinen Formel (I) 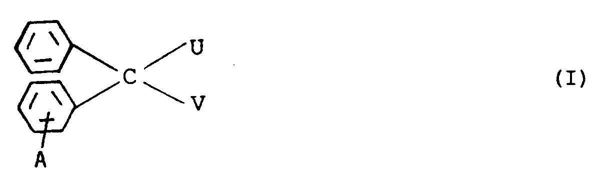 worin
U = H oder W,
V = X-(CH₂)ₙ-Y oder X-W,
A = [(CH₂)ₖ-X-(Q)ₘ-Z]ₘ,
W = (CH₂)ₙ-(Q)ₘ-Z,
X = O, S, NR,
Y = COOR, NR₂,
Q = Crosslinker oder Spacer,
Z = Marker oder Trägerstoff
R = H oder Alkyl mit 1-8 C-Atomen,
n = 1-10,
m = 0 oder 1,
k = 0-4,
bedeuten und der Substituent A nur dann vorhanden ist, wenn U = H und V = X-(CH₂)ₙ-Y ist.

2. Verbindungen der allgemeinen Formel (Ia) 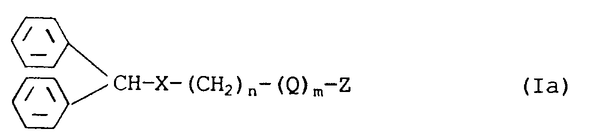 worin
X = O, S, NR,
Q = Crosslinker oder Spacer,
Z = Marker oder Trägerstoff,
R = H oder Alkyl mit 1-4 C-Atomen,
n = 1-10,
m = 0 oder 1
bedeuten.

3. Antikörper erhältlich durch Immunisierung mit einem Immunogen nach den Ansprüchen 1 und 2, worin Z ein Trägerstoff bedeutet.

4. Mittel zur immunologischen Bestimmung von Diphenhydramin und dessen Metaboliten in Körperflüssigkeiten, dadurch gekennzeichnet, daß es
a) ein markiertes Diphenhydraminderivat der allgemeinen Formel (I), worin Z ein Marker bedeutet,
b) Antikörper, die Diphenhydramin, dessen Metaboliten und das markierte Diphenhydraminderivat binden können und
c) gegebenenfalls ein Enzymsubstrat enthält.

5. Mittel nach Anspruch 4, dadurch gekennzeichnet, daß das markierte Diphenhydraminderivat aus einer über einen Crosslinker mit einem Marker verbundenen Verbindung der allgemeinen Formel (I) besteht.

6. Mittel nach Anspruch 4, dadurch gekennzeichnet, daß das Immunogen zur Herstellung der Antikörper aus einer über einen Crosslinker mit einem Trägerstoff verbundenen Verbindung der allgemeinen Formel (I) besteht.

7. Verfahren zur immunologischen Bestimmung von Diphenhydramin und dessen Mataboliten in Körperflüssigkeiten durch Inkubation der zu untersuchenden Probe mit einem markierten Diphenhydraminderivat der allgemeinen Formel (I), worin Z ein Marker bedeutet, und Antikörper, die Diphenhydramin, dessen Metabolite und das markierte Diphenhydraminderivat binden können sowie gegebenenfalls mit einem Enzymsubstrat und Messung der Fluoreszenz oder der Enzymaktivität.
